# EUROPEAN PATENT APPLICATION

(11) **EP 3 459 536 A1**
(43) Date of publication of application: **27.03.2019**
(21) Application number: 17193028.2
(22) Date of filing: 25.09.2017
(51) Int. Cl.: A61K 31/05, A61K 31/352, A61P 25/08, A61P 1/08

(54) **COMPOSITION COMPRISING CANNABINOIDS AND METHOD FOR THE PREPARATION THEREOF**

(71) Applicant: Krotov, Vadym, 3013 AK Rotterdam (NL)
(72) Inventor: KROTOV, Vadym, 3013 AK ROTTERDAM (NL); FERMAN, Alexander Jusupovich, 3013 AK ROTTERDAM (NL)
(74) Representative: Algemeen Octrooi- en Merkenbureau B.V.

(57) **Abstract**

Described is a cannabidiol (CBD) composition comprising at least 50 w/w% cannabidiol and at most 0.2 w/w% tetrahydrocannabinol (THC) and a method for the preparation thereof from cannabis plant material, comprising the steps of conversion of the cannabinoids from the cannabis plant material into the respective salts and complexes thereof, alcoholic extraction of cannabis plant material, acidification of the cannabinoid salts and complexes into the respective cannabinoid acids, decarboxylation under conditions that result in preferential formation of THC as compared to formation of CBD, providing a decarboxylated mixture, incubating the said decarboxylated mixture with a salt and/or a base so as to convert the cannabinoid acids and the cannabinoids into cannabinoid salt and/or complexes, respectively, adding alcohol to a water: alcohol ratio of 30 - 70 : 70 - 30 wherein the cannabinoid salts and complexes dissolve while THC does not dissolve, and contacting the said mixture to an adsorbent or absorbent, allowing THC to bind, resulting in a THC depleted mixture, acidification of the THC depleted mixture resulting in conversion of the cannabinoid salts and complexes into cannabinoid salts, decarboxylation of the cannabinoid acids into their respective cannabinoids, resulting in a decarboxylated THC depleted mixture and recovery of one or more of the cannabinoids from the said decarboxylated THC depleted mixture. Further described is the use of the cannabidiol composition in the preparation of a medicament.

## Description

The invention relates to a composition comprising at least 50 w/w% cannabidiol (CBD) and at most 0.2 w/w% tetrahydrocannabinol (THC) and to a method for the preparation thereof from cannabis, as well as to the use of the composition in the preparation of a medicament.

In the pharmaceutical industry, cannabinoids find application in treatments of numerous conditions, such as for treatment of epilepsy in children, to reduce nausea and vomiting during chemotherapy, treatment of chronic pain and muscle spasms. Cannabinoids can be produced in a chemical manner, or be extracted from the cannabis plant, of which three species can be recognized, *C.sativa, C. indica and* C. *ruderalis.* Extraction is a very cost effective production method of cannabinoids. However, such extracts contain the psychoactive constituent tetrahydrocannabinol (THC) which is not desired in many applications of cannabinoids as a medicament, in particular when children are concerned. To this end, extracts are prepared from Cannabis varieties that produce minimal levels of THC. About 30 varieties are known that produce below 0.2 w/w% THC per 1 w/w% CBD. Such plants may also comprise the cannabinoids in their acid form. Herein, the term THCA stand for the acid of the cannabinoid THC, whereas CBDA stands for the acid of the cannabinoid CBD.

Attractive extracts comprise a low THC content but a high content of CBD and optionally other cannabinoids such as cannabigerol (CBG), The structural formula of CBD is given below.

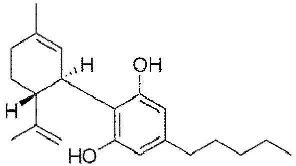

Preparation methods of extracts with reduced THC content are known in the art and involve supercritical CO₂ extraction. However, the extracts obtained by such methods are not consistent in their contents, and therefore less suitable as raw material for processing industries, such as pharmaceutical industries that use cannibinoids as CBD for the preparation of medicaments.

Provided is a novel composition comprising at least 50 w/w% cannabidiol (CBD) and at most 0.2 w/w% tetrahydrocannabinol (THC) and a method for the preparation thereof enabling the provision of the said composition of constant and reliable content consistency. Such composition can also contain about 5 - 10 w/w% pectins, in particular 6 - 8 w/w% pectins, more in particular about 7 w/w% pectins. The term 'about' allows a deviation of the indicated value by 10% (i.e. allowing a range of 6.3 to 7.7 w/w%), preferably of 5%, more preferably of 3% or even 1%. The method for the preparation of the above composition comprises the steps of
a) Providing cannabis plant material comprising cannabinoids and acids thereof,
b) Incubation of the plant material of step a) in an aqueous medium comprising salt and/or hydroxide, under conditions to allow conversion of cannabinoid acids and cannabinoids into the respective cannabinoid salts and/or complexes thereof,
c) Allowing the cannabinoid salts and/or complexes of step b) to dissolve, resulting in an aqueous cannabinoid solution,
d) Mixing the aqueous cannabinoid solution of step c) with an alcohol, resulting in an alcoholic cannabinoid extract,
e) Bringing the water to alcohol ratio of the alcoholic extract of step c) to 30 - 70 : 70 - 30 v/v%, preferably to 40 - 60 : 60 - 40 v/v%, providing an aqueous extract comprising the cannabinoid salts and complexes,
f) Acidification of the aqueous extract of step g) resulting in the cannabinoid salts and complexes to be converted into their acids, producing an aqueous acid extract,
g) Subjecting the cannabinoid acids of step f) to decarboxylation under conditions that result in preferential formation of THC as compared to formation of CBD, providing a decarboxylated mixture,
h) Incubating the decarboxylated mixture of step g) with a salt and/or a base to convert the cannabinoid acids and the cannabinoids into cannabinoid salt and/or complexes, respectively,
i) Adding alcohol to the mixture of step h) to a water: alcohol ratio of 30 - 70 : 70 - 30, preferably of 40 - 60 : 60 - 40, wherein the cannabinoid salts and complexes dissolve while THC does not dissolve,
j) Contacting the mixture of step i) to an adsorbent or absorbent, allowing the THC to bind to the said adsorbent or absorbent, resulting in a THC depleted mixture,
k) Acidification of the THC depleted mixture of step j) resulting in conversion of the cannabinoid salts and complexes in the THC depleted mixture, into cannabinoid acids,
l) Decarboxylation of the cannabinoid acids into their respective cannabinoids, resulting in a decarboxylated THC depleted mixture,
m) Recovery of one or more of cannabinoids from the decarboxylated THC depleted mixture of step I).

In a first step, cannabis plant material is provided. Such plant material comprises different cannabinoids as well as acids thereof. The said plant material may e.g. be cut in smaller pieces or grinded in order to improve the processability thereof. It is advantageous for the plant material to be derived from cannabis varieties that have a low THC content. Such varieties are known in the art as described above. Preferred varieties comprise per 1 w/w% CBD content, at most 0.2 w/w% THC, preferably at most 0.1 w/w% THC, more preferably at most 0.05 w/w% THC, most preferably at most 0.035 w/w% THC. In particular, the female tops of cannabis plants are attractive starting material for the extraction. Such tops have a high CBD content. Therefore, the cannabis plants material is preferably derived from the upper 50 cm of the stems or branches of cannabis plants, where the seeds are growing.

In a subsequent step, the plant material is incubated in an aqueous medium comprising a salt and/or a hydroxide, resulting in salt and complex formation of the cannabinoid, which salts and complexes are soluble in water, in contrast to the cannabinoid. When reacting with the salt provided by the aqueous medium, cannabinoid acid is therewith converted into the corresponding cannabinoid salt. In order to allow the reaction to proceed towards the formation of the cannabinoid salt, a salt may be chosen of an acid that is weaker than CBPA, or a component is formed that precipitates, therewith driving the reaction towards cannabinoid salt formation. In case a carbonate is chosen, CO₂ may be formed by heating the reaction, also driving the reaction towards the envisaged cannabinoid salt formation.

A base, such as potassium hydroxide, reacts with the cannabinoid acid to form a potassium salt.

The cannabinoid tends to form water soluble ligand complexes by reacting with and acid or base. However, it is observed that no complexes are formed when sodium hydroxide or sodium salts are used. It was observed that the salt FeCl₃ results in cannabinoid complex formation, but not in cannabinoid salt formation.

When the complexes and/or salts of the cannabinoids are formed, these will dissolve in the aqueous medium.

By the addition of an alcohol, an alcoholic extract of the cannabinoid salts and complexes is obtained. By this extraction undesired components are removed. Alcoholic extraction of cannabis plants to bring cannabinoids into solution are known in the art. Any alcohol can be used, as long as the CBD dissolves therein in significant amounts. In particular alkanols, more in particular ethanol, but alo isopropanol or butanol can be used. The extraction is preferably done in 70 - 100 v/v% ethanol, where the remainder is water or an aqueous solution, e.g. comprising some salt or a buffer, but is preferably water. In particular, mixtures of 85-95 v/v% ethanol and 5-15 v/v% water are used. However, mixtures of different alcohols, or alcohols with other solvents can be used, optionally in combination with water.

It is to be noted that when potassium carbonate is used as a salt, the above conversion and alcohol extraction can be performed simultaneously, as the aqueous potassium carbonate medium does not mix with the alcohol such as ethanol. During the extraction, a phase separation is obtained, where the cannabinoid salts and complexes reside in the ethanol phase.

Subsequently, the alcohol content is lowered by bringing the ratio of water to alcohol to 30 - 70 : 70 - 30 v/v%, preferably to 40 - 60 : 60 - 40 v/v%, providing an aqueous extract comprising the cannabinoid salts and complexes. This can be done by the addition of water, or by evaporation of alcohol from the extract, or by a combination of both.

The resulting aqueous medium is acidified, e.g. by the addition of a suitable salt, such as e.g. and organic acid, in particular a food grade acid, such as citric acid. The acidification results in the conversion of the cannabinoid salts and complexes into the respective cannabinoid acids.

In a next step, decarboxylation is performed such, that this results in preferential formation of THC from THCA. This can be done by choosing the reaction parameters such, that (1) THCA is decarboxylated into THC, while significantly avoiding the formation of CBD from CBDA, i.e. selective decarboxylation of THCA, or (2) by decarboxylation of both THCA and CBDA, followed by a selective carboxylation of CBD into CBDA while significantly avoiding the formation of THCA from THC.
(1) Selective decarboxylation resulting in decarboxylation of THCA while leaving CBDA substantially untouched can be done by using sodium as catalyst at mild conditions, such as a reaction temperature of 65°C for one hour, or at 70°C or half an hour or 10 minutes at 90°C.
(2) At harsher conditions using sodium as katalyst, both THCA as well as CBDA are decarboxylated to form THC and CBD respectively. When using potassium in a subsequent carboxylation step, CBD is converted into CBDA, while THC remains substantially untouched.

As a result of the above, a decarboxylated mixture is obtained, rich in CBDA, poor in CBD, with a THC content but significantly void of THCA.

In a subsequent step, the decarboxylated mixture is again incubated with a salt and/or a base as described above in order to convert the cannabinoids and cannabinoid acids into salts and complexes. This step is preferably performed in an aqueous medium comprising 40 to 60 v/v% alcohol, in particular ethanol. CBD salts and complexes of CBD will be dissolved in the water phase, whereas THC will be insoluble therein.

The insoluble THC will readily be adsorbed or absorbed to a suitable adsorbent or absorbent, respectively. Suitable adsorbents include Al₂O₃, diatomaceous earth, silicagel, MgO, activated carbon or a combination of two or more thereof. Al₂O₃ is a particular attractive adsorbent for this purpose.

The non-adsorbed material will comprise the CBD salt and complexes, whereas the majority of the THC will be adsorbed. The non-adsorbed material can easily be recovered from the adsorbent material, i.e. by a water or water-alcohol mixture, such as a 1:1 v:v alcohol to water ratio, where the alcohol preferably comprises or even is ethanol.

Subsequently, after removal of the THC by the above-described adsorption, the CBD salts and complexes are converted into CBD acid by acidification of the water-alcohol mixture to a pH of 3 to 6.8, preferably to a pH of 6.0 - 6.5. This can be done by the addition of a suitable acid as described above, in particular citric acid. A subsequent decarboxylation step as discussed above results in CBD.

This CBD enriched and THC depleted preparation can be recovered and further purified by evaporation or drying. Preferably, the enriched cannabidiol preparation thus obtained is recovered, which recovery in particular comprises drying and optionally also washing of the enriched cannabidiol preparation before or after drying with water in order to remove acid and other residues from the preparation to provide the envisaged composition, which appear to reproducibly comprise at least 50 w/w% cannabidiol (CBD) and less than 0.2 w/w% tetrahydrocannabinol (THC), rendering the said composition highly suitable as raw material for the preparation of a medicament.

## Claims

1. Composition, comprising at least 50 w/w% cannabidiol (CBD) and less than 0.2 w/w% tetrahydrocannabinol (THC).

2. Composition according to claim1, further comprising 5 - 10 w/w% pectins.

3. Method for the preparation of a composition according to claim 1 or 2 from cannabis plant material, comprising the steps of:
a) Providing cannabis plant material comprising cannabinoids and acids thereof,
b) Incubation of the plant material of step a) in an aqueous medium comprising salt and/or hydroxide, under conditions to allow conversion of cannabinoid acids and cannabinoids into the respective cannabinoid salts and/or complexes thereof,
c) Allowing the cannabinoid salts and/or complexes of step b) to dissolve, resulting in an aqueous cannabinoid solution,
d) Mixing the aqueous cannabinoid solution of step c) with an alcohol, resulting in an alcoholic cannabinoid extract,
e) Bringing the water to alcohol ratio of the alcoholic extract of step c) to 30 - 70 : 70 - 30 v/v%, preferably to 40 - 60 : 60 - 40 v/v%, providing an aqueous extract comprising the cannabinoid salts and complexes,
f) Acidification of the aqueous extract of step g) resulting in the cannabinoid salts and complexes to be converted into their acids, producing an aqueous acid extract,
g) Subjecting the cannabinoid acids of step f) to decarboxylation under conditions that result in preferential formation of THC as compared to formation of CBD, providing a decarboxylated mixture,
h) Incubating the decarboxylated mixture of step g) with a salt and/or a base to convert the cannabinoid acids and the cannabinoids into cannabinoid salt and/or complexes, respectively,
i) Adding alcohol to the mixture of step h) to a water: alcohol ratio of 30 - 70 : 70 - 30, preferably of 40 - 60 : 60 - 40, wherein the cannabinoid salts and complexes dissolve while THC does not dissolve,
j) Contacting the mixture of step i) to an adsorbent or absorbent, allowing the THC to bind to the said adsorbent or absorbent, resulting in a THC depleted mixture,
k) Acidification of the THC depleted mixture of step j) resulting in conversion of the cannabinoid salts and complexes in the THC depleted mixture, into cannabinoid acids,
l) Decarboxylation of the cannabinoid acids into their respective cannabinoids, resulting in a decarboxylated THC depleted mixture,
m) Recovery of one or more of cannabinoids from the decarboxylated THC depleted mixture of step I).

4. Method for the preparation of a composition according to claim 3, wherein the cannabis plant material in step a) is derived from a cannabis variety that comprises, per 1 w/w% CBD content, at most 0.2 w/w% THC, preferably at most 0.1 w/w% THC, more preferably at most 0.05 w/w% THC, most preferably at most 0.035 w/w% THC.

5. Method for the preparation of a composition according to claim 3 or 4, wherein the cannabis plant material is derived from the upper 50 cm of the stems and branches of cannabis plants.

6. Method for the preparation of a composition according to any of the claims 3 - 5, wherein in step d) is performed in 70 - 100 v/v% ethanol and 30 - 0 v/v% water, preferably in 85 - 95 v/v% ethanol and 5 - 15 v/v% water.

7. Method for the preparation of a composition according to any of the claims 3 - 7, wherein the salt in the aqueous medium of step b) comprises carbonate, wherein the carbonate is preferably chosen from sodium carbonate and potassium carbonate or a mixture thereof.

8. Method for the preparation of a composition according to any of the claims 3 - 7, wherein in step e) the alcohol is evaporated to the envisaged water to alcohol ratio.

9. Method for the preparation of a composition according to any of the claims 3 - 8, wherein the decarboxylation of step g) is between 60 and 65°C for 30 minutes to 2 hours.

10. Method for the preparation of a composition according to any of the claims 3 - 9, wherein the salt in step h) comprises potassium carbonate and/or potassium hydrogen carbonate.

11. Method for the preparation of a composition according to any of the claims 3 - 9, wherein the adsorbent/absorbent in step j) comprises an alkali adsorbent/absorbent, the adsorbent/absorbent preferably comprising Al₂O₃, diatomaceous earth, silicagel, MgO, activated carbon or a combination of two or more thereof.

12. Method for the preparation of a composition according to any of the claims 3 -10, wherein the acidification in step f) and/or k) comprises addition of citric acid.

13. Method for the preparation of a composition according to any of the claims 3 - 11, wherein step m) comprises evaporation and/or drying the cannabinoid.

14. Method for the preparation of a composition according to any of the claims 3 - 12, further comprising a step n) of washing the recovered material of step m) with water, optionally followed by a drying step.

15. Use of the composition of claim 1 or 2 for the preparation of a medicament.
